# FASCICULE DE BREVET EUROPEEN

(11) **EP 1 675 513 B1**
(45) Date de publication et mention de la délivrance du brevet: **27.02.2013**
(21) Numéro de dépôt: 04805289.8
(22) Date de dépôt: 22.10.2004
(51) Int. Cl.: A61B 17/70

(54) **SUPPORT INTER-LAMAIRE**
KLINGEN-ZWISCHENSTÜTZE
INTER-BLADE SUPPORT

(30) Priorité: 24.10.2003 FR 0312487
(43) Date de publication de la demande: 05.07.2006
(73) Titulaire: Cousin Biotech, S.A.S., F-59117 Werwicq Sud (FR); Smart Hospital S.R.L., 55060 San Martino in Freddana (IT)
(72) Inventeur: PETRINI, Piero, I-06100 Perugia (IT); DENEUVILLERS, Guy, F-62155 Merlimont (FR)
(74) Mandataire: Balesta, Pierre
(86) Numéro de dépôt international: PCT/FR2004/002727
(87) Numéro de publication internationale: WO 2005/044118

(56) Documents cités:
- WO-A-2004/084743
- FR-A- 2 717 675
- JP-A- 09 075 381
- US-A- 5 496 318
- US-A1- 2001 016 743
- PATENT ABSTRACTS OF JAPAN vol. 2003, no. 07, 3 juillet 2003 (2003-07-03) & JP 2003 079649 A (PENTAX CORP;HASE HITOSHI), 18 mars 2003 (2003-03-18)

## Description

La présente invention se rapporte au domaine technique des prothèses vertébrales destinées à redistribuer, entre deux vertèbres adjacentes, la surcharge créée par la dégénérescence du disque, sans immobiliser les mouvements articulaires, et laissant la possibilité de suivre les mouvements du rachis.

Les prothèses comprenant une partie en matière déformable sont déjà connues. Dans le brevet FR2623085 au nom de Francis Bréard, est décrit une sorte de cale ayant deux extrémités et étant apte à être insérée entre les apophyses épineuses de deux vertèbres adjacentes. La cale est retenue au moyen de ligaments passant dans des trous latéraux.

Une prothèse d'un concept quasi similaire est décrite dans le brevet européen 0322334, inventeur Jean-Jacques Bronsard. Il est décrit qu'un ou plusieurs coussinets cylindriques élastiques et creux sont interposés entre les apophyses épineuses de deux vertèbres adjacentes et sont fixés au moyen d'un ligament qui les traverse. D'autres prothèses inter-épineuses de forme diverse sont décrites dans les brevets FR2717675 et FR2775183 du Dr Jean Taylor et dans JP 090 75381 qui décrit les caractéristiques trouvées dans le préamble de la revendication 1.

Bien que ces dispositifs déjà connus fournissent des résultats intéressants en matière d'écart discal, par une fixation entre les apophyses épineuses, ils souffrent néanmoins d'inconvénients non négligeables puisqu'ils n'offrent pas une récupération de la charge des efforts appropriée aux besoins physiologiques. L'absorption de la charge transmise entre les vertèbres reste jusqu'à présent partielle.

De telles prothèses étant interposées entre les apophyses épineuses sont décentrées par rapport au barycentre des corps vertébraux, sur lequel est supportée la charge maximum, alors que la grande partie des charges passent par un axe situé au centre des corps vertébraux.

Le premier désavantage de ces dispositifs connus est que seule une partie de cette charge est absorbée par la prothèse, ce qui ne permet pas d'avoir une fonction amortissante pleinement efficace.

Le deuxième désavantage est que la mobilité articulaire de ce type de prothèse est réduite, le contrôle complet de la flexion, de l'extension et de la rotation du rachis étant alors substantiellement limité.

Le troisième désavantage est que ces dispositifs connus sont tous invasifs, puisque l'on doit procéder à l'ablation du ligament postérieur sain ou la détérioration des muscles latéraux adjacents pour pouvoir les placer.

L'implant vertébral selon la présente invention remédie à de tels inconvénients par ses matériaux, son aspect fonctionnel et ses formes spécifiquement adaptés à un amortissement efficace au plus près du canal vertébral. La présence de corps élastiques souples destinés à être insérés entre deux vertèbres adjacentes, dans l'espace entre les lames sus et sous jacentes du niveau instrumenté, stabilise le support en antérieur et en postérieur par des moyens de retenue intégrés.

Un des nombreux avantages du support inter-lamaire est de ramener le point d'appui au niveau de l'arc postérieur, là où la charge est la plus importante.

En effet le point d'appui de l'unité fonctionnelle, sur lequel est concentrée la charge encaissée lors des mouvements du rachis, se positionne graduellement vers la partie postérieure du canal médullaire et se situe exactement dans la zone inter-lamaire à l'abri des facettes articulaires des vertèbres, au plus proche de l'axe médullaire de répartition des forces encaissées lors des mouvements de la colonne.

La distance entre l'axe dudit corps élastique et le barycentre des corps vertébraux est alors substantiellement réduite, en comparaison des dispositifs connus décrits ci-avant.

Une autre fonction avantageuse de cet implant inter-lamaire est de redonner et de maintenir un écart discal satisfaisant et de mieux amortir les efforts exercés sur le niveau, offrant un meilleur soulagement du disque intervertébral.

Les constituants et formes du support inter-lamaire permettent une apposition entre les lames des vertèbres de la partie postérieure de la colonne vertébrale, tout particulièrement aux niveaux dorso-lombaire, lombaire et lombosacrée, sa forme s'adaptant aux variations anatomiques des niveaux concernés.

La souplesse et la flexibilité du matériau dans lequel est réalisé le support inter-vertébral permettent de conserver un point d'articulation permettant une mobilité tridimensionnelle tout en donnant un espace intervertébral anatomique stable.

Le faible encombrement permet de réduire les ablations de ligaments et muscles sains tenant les facettes articulaires. Lors de la pose, l'espace de préparation se limitera à un cadre minimal acceptant l'appui de l'implant entre les lames du niveau instrumenté, et laissant un maximum de tissus intact. L'implant est micro-invasif.

L'invention consiste en un support intervertébral permettant de maintenir un écart intervertébral anatomique et restituer la mobilité tridimensionnelle du niveau instrumenté, comprenant une cale et des moyens de retenue. L'invention comprend deux zones, définies dans la partie caractérisante de la revendication. 1

Une partie postérieure assure la mobilité et l'amortissement au niveau instrumenté. Celle-ci comprend des moyens de retenue ayant pour fonction de bloquer la migration du support vers la partie antérieure du rachis, en s'appuyant contre les lames. Une partie antérieure, apte à se loger entre les lames des vertèbres, redonne un écart intervertébral anatomique.

Des moyens de retenue, constitués d'épaulements latéraux, de saillies transversales sur les faces supérieure et inférieure de l'implant, et des gouttières moulées dans la partie antérieure, permettent de le caler en le maintenant en butée et en appui à la jonction des lames et des épineuses. Cela permet de bloquer la migration du support vers la partie antérieure du rachis.

Les épaulements latéraux de la partie postérieure peuvent être constitués de larges surfaces symétriquement opposées, en retrait de la partie antérieure, aptes à se loger contre les lames des vertèbres au plus près des facettes articulaires. Les épaulements peuvent également être de faible surface, à type bourrelets débordants, symétriquement opposés en retrait de la partie antérieure, aptes à libérer le mouvement des facettes articulaires vertébrales.

Les épaulements latéraux ne dépassent pas la hauteur la plus importante de la partie postérieure du support et sont de faible largeur, par rapport support pris dans son ensemble.

La partie postérieure comprend une face inférieure venant porter sur la partie supérieure de l'épineuse inférieure.

Cette partie postérieure, permettant d'amortir les mouvements entre deux vertèbres adjacentes, est réalisée, dans une variante de conception, en une forme prismatique de hauteur correspondant à l'écart entre les vertèbres adjacentes, dont au moins un angle est arrondi, la face supérieure de la partie postérieure de la cale étant de forme triangulaire, afin de recevoir le point de jonction formé par la lame et l'épineuse. Cette forme donnant une stabilité entre les vertèbres sus et sous jacentes dudit niveau instrumenté.

Dans une autre forme de conception, la partie postérieure autorise une liberté de mouvement entre la face supérieure de la cale et l'épineuse supérieure du niveau instrumenté, grâce à sa forme fuyante.

La partie postérieure du dispositif présente des surfaces supérieure et inférieure évasées dans leur partie antérieure, jusqu'aux saillies transversales, s'affinant progressivement vers la partie postérieure extrême desdites surfaces, et recevant le point de jonction formé par la lame et l'épineuse.

Le coeur de la partie postérieure peut être traversé de part en part par un évidement, permettant d'augmenter l'élasticité de l'ensemble.

Le coeur de la partie postérieure peut supporter des dents espacées de sillons, opposés un à un, sur les faces inférieure et supérieure, permettant de faire varier l'élasticité de l'ensemble.

La partie verticale des épaulements en contact avec les lames présente une zone suffisamment concave se prolongeant dégressivement vers la zone latérale postérieure du dispositif, afin de libérer l'espace des facettes articulaires.

Le matériau permettant de définir le module d'élasticité est de la silicone, d'une dureté de 40 à 80 Shore A. Elle permet de définir le module d'élasticité adapté aux contraintes, tout en autorisant, au moins partiellement, une liberté de mobilité au niveau instrumenté. Au moins la partie postérieure est constituée de silicone.

Dans une variante de conception, l'invention comporte des moyens de retenue additionnels constitués par des ligaments et, le cas échéant, des perçages dans la hauteur de l'implant pour leur passage. Ces ligaments peuvent être autonomes ou croisés et traversent le support dans toute sa largeur ou sa hauteur. Des perçages sont prévus pour permettre leur passage.

Le support peut être enrobé en partie d'un tissu biocompatible à maille, laissant la partie antérieure à nu pour éviter la fibrose du côté des tissus nobles.

La partie antérieure du support intègre en son coeur une boucle en matériau biocompatible rigide. Dans une variante de conception, la partie antérieure est intégralement constituée d'un matériau biocompatible rigide.

La partie postérieure présente en son milieu et dans sa longueur une gorge de faible profondeur, apte entrer en contact avec l'épineuse supérieure du niveau instrumenté.

Les dessins ci joints, donnés à titre d'exemple indicatif et non limitatif, seront plus explicites :

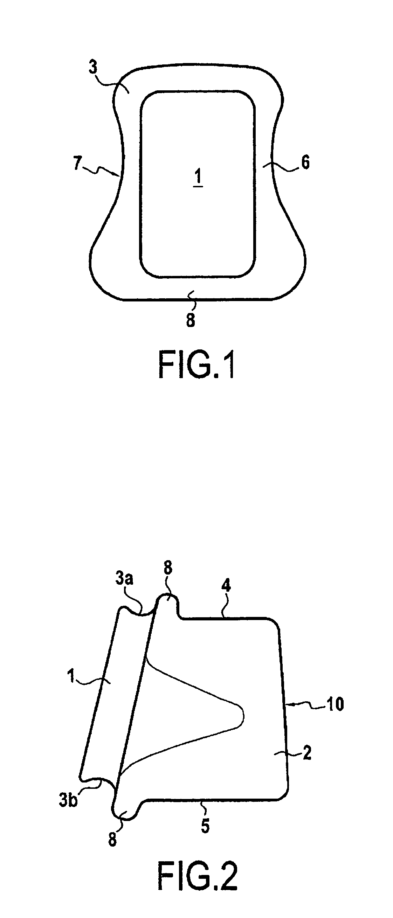

La figure 1 présente une vue en bout de la face antérieure de l'invention.
La figure 2 est une vue de côté.

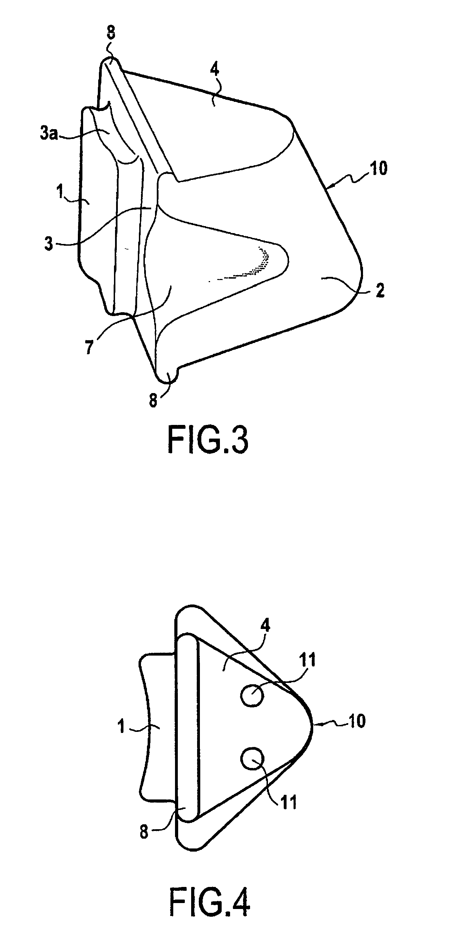

La figure 3 est une vue en perspective.
La figure 4 présente une vue de dessus dans laquelle l'implant présente des passages pour les ligaments.

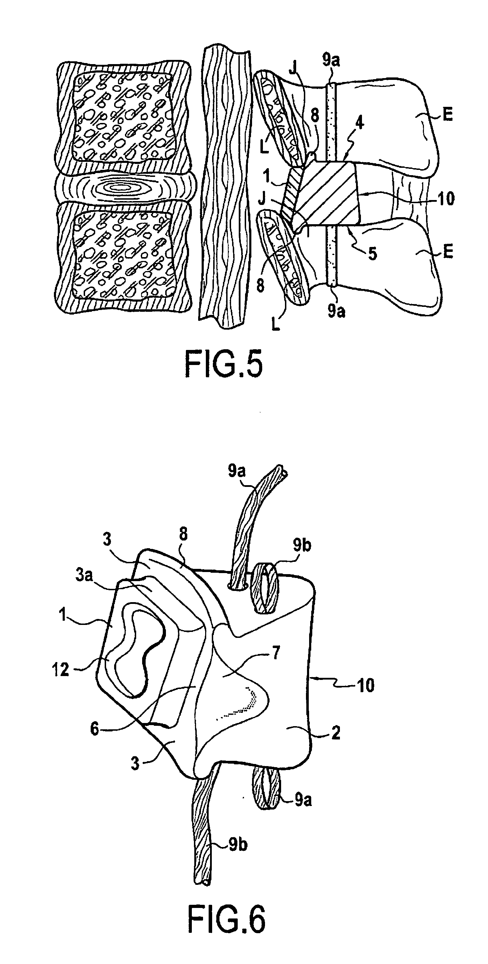

La Figure 5 est une vue de l'implant conforme à l'invention, muni de ligaments, après mise en place entre deux vertèbres.
La figure 6 est une vue en perspective.

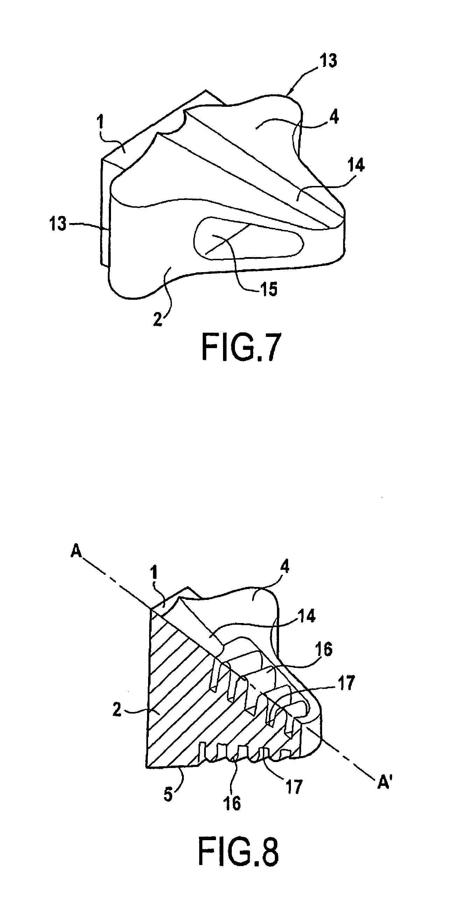

La figure 7 présente une vue en perspective.
La figure 8 est une vue en perspective coupée selon un plan AA'.

Dans un mode de réalisation, le support est en silicone, d'une dureté comprise entre 40 et 80 shore A, ou en polyéthylène au niveau des appuis lamaires.

Le support est réalisé en matériau biocompatible, autorisant une certaine mobilité dans tous les axes, afin de s'adapter aux mouvements complexes des vertèbres. La silicone permet de varier l'effet amortissant de l'implant. L'injection moulage de silicone de grade médical, implantable à plus de trente jours, permet d'obtenir un tel support.

Dans un mode préférentiel de réalisation, l'implant est obtenu par un surmoulage de silicone autour d'une boucle (12), en polyétherétherkétone ou métal biocompatible, disposée au centre de la partie antérieure (1).

Idéalement le support est incorporé et tient de lui-même entre l'axe médullaire et articulaire de la colonne, au plus près possible du canal médullaire sans être au contact de la dure mère. La face antérieure (1) du support reste en silicone nu, afin d'éviter la fibrose, l'implant pouvant alors dans ce cas être proche de la dure mère. Le reste du support ou implant est recouvert d'un tissu biocompatible à maille.

Dans un mode particulier de réalisation, la partie postérieure (2) du support est prismatique, et comprend une surface d'appui (3), en butée sur les lames (L), débordant la partie antérieure (1), afin d'éviter la possibilité d'un déplacement de cette dernière vers le canal médullaire.

La face supérieure (4) de la partie postérieure de la cale est évasée, afin de recevoir le point de jonction (J) formé par la lame (L) et l'épineuse (E). La face inférieure (5) vient porter sur la partie supérieure de l'épineuse (E) inférieure.

L'arête (10) de la partie postérieure (2) possède un angle arrondi. La partie verticale (6) de la surface d'appui (3) présente une zone suffisamment concave (7) pour libérer l'espace des facettes articulaires.

Les moyens de retenue, adaptés à l'espace inter-lamaire et permettant une adaptation optimum de l'implant comprennent deux saillies transversales (8) moulées dans le corps de silicone, l'une sur la face supérieure (4) de l'implant, l'autre sur la face inférieure de l'implant (5), deux gouttières (3a et 3b) sur la partie antérieure (1). Dans des modes alternatifs de réalisation, les moyens de retenue, ou épaulements latéraux, sont de larges surfaces symétriquement opposées (13) en retrait de la partie antérieure.

L'implant peut être ainsi positionné sans ligament au niveau de la jonction des lames et des épineuses (J). Le chirurgien aura préalablement créé deux entailles dans ces dernières, pour accueillir les saillies et bloquer ainsi le déplacement du support en postérieur.

Dans une variante de conception, le coeur de la partie postérieure (2) est traversé de part en part par un évidement (15), permettant d'augmenter l'élasticité de l'ensemble.

Dans une autre variante de conception, le coeur de la partie postérieure supporte des dents (16) espacées de sillons (17) opposés un à un, sur les faces inférieure et supérieure, permettant de faire varier l'élasticité de l'ensemble.

Dans une variante de conception, deux ligaments croisés (9a, 9b) traversent le support en son milieu, des perçages (11) étant prévus dans sa hauteur. Le premier ligament est fixé à la boucle du second ligament, située à la base du support, et inversement. Ils entourent chacun une des épineuses sus et sous jacentes au niveau instrumenté.

Une autre variante de conception consiste à ce que soit moulée sur la face supérieure (4) de la partie postérieure (2), en son milieu et dans sa longueur, une gorge de faible profondeur (14).

Il va de soi que de nombreuses variantes peuvent être mise en oeuvre, notamment par substitution de moyens analogues, sans pour autant sortir du cadre de l'invention.

## Revendications

1. Support intervertébral adapté notamment pour permettre de retrouver et maintenir un écart intervertébral anatomique entre les lames supérieure et inférieure (L) de deux vertèbres adjacentes, et restituer la mobilité tridimensionnelle du niveau instrumenté, ledit support Intervertébral comportant une partie antérieure (1) et une partie postérieure (2),
**caractérisé en ce que** la partie antérieure (1) ayant pour fonction de redonner un écart intervertébral anatomique, est munie de deux gouttières (3a, 3b) aptes à recevoir respectivement la partie inférieure de la lame supérieure et la partie supérieure de la lame inférieure pour se positionner dans l'espace entre les lames supérieure et inférieure (L) des deux vertèbres adjacentes, et **en ce que** la partie postérieure (2) comprend des moyens de retenue (3, 8, 13) présentant une surface d'appui (3) en butée sur les lames (L) et débordant la partie antérieure (1) en sorte de bloquer la migration du support vers la partie antérieure du rachis.

2. Support selon la revendication 1, **caractérisé en ce que** les moyens de retenue (3, 8, 13) consistent notamment en des épaulements latéraux (13) en retrait de la partie antérieure (1) et aptes à se loger contre les lames (L) des vertèbres au plus près des facettes articulaires.

3. Support selon la revendication 2, **caractérisé en ce que** les épaulements latéraux (13) sont de faible surface du type bourrelets débordants, symétriquement opposés, en retrait de la partie antérieure (1), et sont aptes à libérer le mouvement des facettes articulaires.

4. Support selon les revendications 2 ou 3, **caractérisé en ce que** les épaulements latéraux (13) ne dépassent pas la hauteur la plus Importante de la partie postérieure (2) du support et sont de faible largeur.

5. Support selon l'une quelconque des revendications précédentes, **caractérisé en ce que** les moyens de retenue (3, 8, 13) comportent deux saillies transversales (8) dont l'une se situe sur la face supérieure (4) du support, et l'autre sur la face inférieure (5) du support.

6. Support selon l'une quelconque des revendications précédentes, **caractérisé en ce que** la partie postérieure (2) est apte à permettre d'amortir les mouvements entre les deux vertèbres adjacentes.

7. Support selon l'une quelconque des revendications précédentes, **caractérisé en ce que** la partie postérieure (2) comprend une face inférieure (5) venant porter sur la partie supérieure de l'épineuse (E) inférieure du niveau instrumenté.

8. Support selon l'une quelconque des revendications précédentes, **caractérisé en ce que** la partie postérieure (2) est de forme prismatique et de hauteur correspondant à l'écart entre les vertèbres adjacentes, présentant au moins un angle arrondi, et une face supérieure (4) de forme triangulaire, afin de recevoir le point de jonction (J) formé par la lame (L) et l'épineuse (E).

9. Support selon l'une des revendications 1 à 8, **caractérisé en ce que** la partie postérieure (2) présente une forme fuyante pour autoriser une liberté de mouvement entre la face supérieure du support et l'épineuse (E) supérieure du niveau instrumenté.

10. Support selon l'une quelconque des revendications précédentes, **caractérisé en ce que** la partie postérieure (2) présente une face supérieure (4) et une face inférieure (5) évasées dans l'extrémité antérieure du support, s'affinant progressivement vers l'extrémité postérieure desdites faces (4 et 5), et recevant le point de jonction (J) formé par la lame (L) et l'épineuse (E).

11. Support selon l'une quelconque des revendications précédentes, **caractérisé en ce que** le coeur de la partie postérieure (2) est traversé de part en part par un évidement (15), permettant d'augmenter l'élasticité de l'ensemble.

12. Support selon l'une des revendications 1 à 11, la partie postérieure (2) comportant une face supérieure (4) et une face inférieure (5), **caractérisé en ce que** le coeur de la partie postérieure (2) supporte des dents (16) espacées de sillons (17), opposés un à un sur les faces inférieure (5) et supérieure (4), permettant de faire varier l'élasticité de l'ensemble.

13. Support selon l'une quelconque des revendications précédentes rattachée à la revendication 2, **caractérisé en ce que** les parties verticales des épaulements latéraux (13) en contact avec les lames (L) présentent chacune une zone concave (7) se prolongeant dégressivement vers la zone latérale postérieure.

14. Support selon l'une quelconque des revendications précédentes, **caractérisé en ce qu'**au moins la partie postérieure (2) est composée de silicone d'une dureté comprise entre 40 et 80 shore A, autorisant une liberté de mobilité au niveau instrumenté et une élasticité afin de rétablir la lordose.

15. Support selon l'une quelconque des revendications précédentes, **caractérisé en ce que** la partie postérieure (2) est recouverte au moins partiellement par un tissu biocompatible à maille.

16. Support selon l'une quelconque des revendications précédentes, **caractérisé en ce que** la partie antérieure (1) intègre en son centre une boucle (12) en un matériau rigide biocompatible.

17. Support selon l'une quelconque des revendications précédentes, **caractérisé en ce que** la partie antérieure (1) est intégralement constituée d'un matériau rigide biocompatible.

18. Support selon l'une quelconque des revendications précédentes, **caractérisé en ce qu'**il comporte des moyens de retenue supplémentaires constitués par des ligaments (9a, 9b) se croisant au centre dudit support, et des perçages (11) dans sa hauteur pour le passage des ligaments.

19. Support selon l'une des revendications 1 à 18, **caractérisé en ce qu'**il comporte des moyens de retenue supplémentaire constitués par des ligaments autonomes traversant le support dans toute sa largeur.

20. Support selon l'une quelconque des revendications précédentes, **caractérisé en ce que** la partie postérieure (2) comporte une face supérieure (4) présentant en son milieu et dans sa longueur une gorge (14) de faible profondeur apte entrer en contact avec l'épineuse supérieure du niveau instrumenté.

## Claims

1. An intervertebral support adapted in particular to restoring and maintaining an anatomical intervertebral spacing between the top and bottom laminae (L) of two adjacent vertebrae, and to restoring three-dimensional mobility where the support is installed, said intervertebral support comprising an anterior portion (1) and a posterior portion (2),
the support being **characterized in that** the anterior portion (1) having the function of restoring an anatomical intervertebral spacing, is provided with two grooves (3a, 3b) respectively suitable for receiving the bottom portion of the top lamina and the top portion of the bottom lamina in order to be positioned in the space between the top and bottom laminae (L) of the two adjacent vertebrae, and **in that** the posterior portion (2) comprises retaining means (3, 8, 13) presenting a bearing surface (3) in abutment against the laminae (L) and projecting from the anterior portion (1) so as to prevent the support from migrating towards the anterior portion of the spine

2. A support according to claim 1, **characterized in that** the retaining means (3, 8, 13) particularly consist in lateral shoulders (13) set back from the anterior portion (1) and suitable for being received against the laminae (L) of the vertebrae as close as possible to the articular facets.

3. A support according to claim 2, **characterized in that** the lateral shoulders (13) are of small area being of the type having symmetrically-opposite projecting bulges set back from the anterior portion (1) and suitable for releasing movement of the vertebral articular facets.

4. A support according to claim 2 or claim 3, **characterized in that** the lateral shoulders (13) present height that does not exceed the height of the posterior portion (2) of the support, and are narrow in width.

5. A support according to any preceding claim, **characterized in that** the retaining means (3, 8, 13) include two transverse projections (8), one of which is located on the top face (4) of the support and the other on the bottom face (5) of the support.

6. A support according to any preceding claim, **characterized in that** the posterior portion (2) is suitable for serving to damp movements between the two adjacent vertebrae.

7. A support according to any preceding claim, **characterized in that** the posterior portion (2) comprises a bottom face (5) bearing on the top portion of the process (E) at the bottom of the region fitted with the implant.

8. A support according to any preceding claim, **characterized in that** the posterior portion (2) is prismatic in shape and of a height that corresponds to the spacing between the adjacent vertebrae, presenting at least one rounded corner, and a top face (4) that is triangular in shape, so as to receive the junction point (J) formed by the lamina (L) and the processes (E).

9. A support according to any one of claims 1 to 8, **characterized in that** the posterior portion (2) presents a tapering shape so as to allow freedom of movement between the top face of the support and the process (E) above the region fitted with the implant.

10. A support according to any preceding claim, **characterized in that** the posterior portion (2) presents a top face (4) and a bottom face (5) that are flared to the anterior end of the support, tapering progressively towards the posterior ends of said faces (4 and 5), and receiving the junction point (J) formed by the lamina (L) and the process (E).

11. A support according to any preceding claim, **characterized in that** the core of the posterior portion (2) is pierced by a through recess (15), enabling the flexibility of the implant to be increased.

12. A support according to any one of claims 1 to 11, the posterior portion (2) comprising a top face (4) and a bottom face (5), the support being **characterized in that** the core of the posterior portion (2) carries teeth (16) spaced apart by furrows (17), and opposed to each other in pairs on the bottom and top faces (5 and 4), enabling the flexibility of the assembly to be varied.

13. A support according to any preceding claim as dependent on claim 2, **characterized in that** the vertical portions of the lateral shoulders (13) in contact with the laminae (L) present respective concave zones (7) extending and tapering towards the posterior lateral zone.

14. A support according to any preceding claim, **characterized in that** at least the posterior portion (2) is made of silicone having hardness lying in the range 40 to 80 on the Shore A scale, allowing freedom of movement in the region fitted with the implant, and flexibility in order to relieve lordosis.

15. A support according to any preceding claim, **characterized in that** at least part of the posterior portion (2) is covered with a biocompatible knit fabric.

16. A support according to any preceding claim, **characterized in that** the anterior portion (1) has a loop (12) of rigid biocompatible material in its center.

17. A support according to any preceding claim, **characterized in that** the anterior portion (1) is constituted entirely out of rigid biocompatible material.

18. A support according to any preceding claim, **characterized in that** it includes additional retention means constituted by ligaments (9a, 9b) crossing in the center of said support, and holes (11) extending vertically for passing the ligaments.

19. A support according to any one of claims 1 to 18, **characterized in that** it includes additional retaining means constituted by independent ligaments passing through the full height of the support.

20. A support according to any preceding claim, **characterized in that** the posterior portion (2) includes a top face (4) that presents a shallow groove (14) extending lengthwise in its middle and suitable for coming into contact with the process above the region fitted with the implant.

## Patentansprüche

1. Intervertebralstütze, die insbesondere dazu ausgelegt ist, zu ermöglichen, einen anatomischen Zwischenwirbelabstand zwischen der oberen und der unteren Platte (L) von zwei benachbarten Wirbeln wiederzuerhalten und aufrechtzuerhalten und die dreidimensionale Beweglichkeit des instrumentierten Bereichs wieder herzustellen, wobei die Intervertebralstütze einen vorderen Teil (1) und einen hinteren Teil (2) umfaßt,
**dadurch gekennzeichnet, daß** der vordere Teil (1), dessen Funktion darin besteht, wieder einen anatomischen Zwischenwirbelabstand zu verleihen, mit zwei Rinnen (3a, 3b) versehen ist, die geeignet sind, den unteren Teil der oberen Platte bzw. den oberen Teil der unteren Platte aufzunehmen, um sich in dem Raum zwischen der oberen und der unteren Platte (L) der beiden benachbarten Wirbel zu positionieren, und daß der hintere Teil (2) Haltemittel (3, 8, 13) umfaßt, die eine Anlagefläche (3) aufweisen, welche an den Platten (L) in Anschlag ist und über den vorderen Teil (1) hinausragt, um das Wandern der Stütze zum vorderen Teil der Wirbelsäule zu hemmen.

2. Stütze nach Anspruch 1, **dadurch gekennzeichnet, daß** die Haltemittel (3, 8, 13) insbesondere aus seitlichen Schultern (13) bestehen, die gegenüber dem vorderen Teil (1) zurückliegen und geeignet sind, sich an die Platten (L) der Wirbel möglichst nahe den Gelenkflächen anzulegen.

3. Stütze nach Anspruch 2, **dadurch gekennzeichnet, daß** die seitlichen Schultern (13) von geringer Fläche vom Typ überstehende Wülste, symmetrisch gegenüberliegend, gegenüber dem vorderen Teil (1) zurückliegend sind und geeignet sind, die Bewegung der Gelenkflächen freizugeben.

4. Stütze nach den Ansprüchen 2 oder 3, **dadurch gekennzeichnet, daß** die seitlichen Schultern (13) die größte Höhe des hinteren Teils (2) der Stütze nicht überschreiten und eine geringe Breite aufweisen.

5. Stütze nach einem der vorhergehenden Ansprüche, **dadurch gekennzeichnet, daß** die Haltemittel (3, 8, 13) zwei Quervorsprünge (8) umfassen, von denen einer sich an der Oberseite (4) der Stütze und der andere sich an der Unterseite (5) der Stütze befindet.

6. Stütze nach einem der vorhergehenden Ansprüche, **dadurch gekennzeichnet, daß** der hintere Teil (2) geeignet ist, das Dämpfen der Bewegungen zwischen den beiden benachbarten Wirbeln zu ermöglichen.

7. Stütze nach einem der vorhergehenden Ansprüche, **dadurch gekennzeichnet, daß** der hintere Teil (2) eine Unterseite (5) aufweist, die auf dem oberen Teil des unteren Dorns (E) des instrumentierten Bereichs ruht.

8. Stütze nach einem der vorhergehenden Ansprüche, **dadurch gekennzeichnet, daß** der hintere Teil (2) eine Prismenform und eine dem Abstand zwischen den benachbarten Wirbeln entsprechende Höhe, mit wenigstens einer abgerundeten Ecke, sowie eine dreieckige Oberseite (4) aufweist, um die durch die Platte (L) und den Dorn (E) gebildete Verbindungsstelle (J) aufzunehmen.

9. Stütze nach einem der Ansprüche 1 bis 8, **dadurch gekennzeichnet, daß** der hintere Teil (2) eine sich verjüngende Form aufweist, um eine Bewegungsfreiheit zwischen der Oberseite der Stütze und dem oberen Dorn (E) des instrumentierten Bereichs zuzulassen.

10. Stütze nach einem der vorhergehenden Ansprüche, **dadurch gekennzeichnet, daß** der hintere Teil (2) eine Oberseite (4) und eine Unterseite (5) aufweist, die im vorderen Ende der Stütze erweitert sind, sich zum hinteren Ende der Seiten (4 und 5) schrittweise verschmälern und die durch die Platte (L) und den Dorn (E) gebildete Verbindungsstelle (J) aufnehmen.

11. Stütze nach einem der vorhergehenden Ansprüche, **dadurch gekennzeichnet, daß** der Kern des hinteren Teils (2) vollkommen von einer Ausnehmung (15) durchzogen ist, die ermöglicht, die Elastizität der Anordnung zu erhöhen.

12. Stütze nach einem der Ansprüche 1 bis 11, wobei der hintere Teil (2) eine Oberseite (4) und eine Unterseite (5) umfaßt, **dadurch gekennzeichnet, daß** der Kern des hinteren Teils (2) an der Unterseite (5) und der Oberseite (4) durch einzeln gegenüberliegende Rillen (17) beabstandete Zähne (16) trägt, die ermöglichen, die Elastizität der Anordnung zu verändern.

13. Stütze nach einem der vorhergehenden Ansprüche, in Verbindung mit Anspruch 2, **dadurch gekennzeichnet, daß** die vertikalen Teile der seitlichen Schultern (13), die mit den Platten (L) in Kontakt sind, jeweils einen konkaven Bereich (7) aufweisen, der sich in Richtung des hinteren Seitenbereichs abnehmend fortsetzt.

14. Stütze nach einem der vorhergehenden Ansprüche, **dadurch gekennzeichnet, daß** wenigstens der hintere Teil (2) aus Silikon mit einer Härte im Bereich zwischen 40 und 80 Shore A besteht, das eine Bewegungsfreiheit im instrumentierten Bereich sowie eine Elastizität zuläßt, um die Lordose wiederherzustellen.

15. Stütze nach einem der vorhergehenden Ansprüche, **dadurch gekennzeichnet, daß** der hintere Teil (2) wenigstens teilweise mit einem biokompatiblen Maschengewebe überzogen ist.

16. Stütze nach einem der vorhergehenden Ansprüche, **dadurch gekennzeichnet, daß** der vordere Teil (1) in seiner Mitte eine Öse (12) aus einem starren, biokompatiblen Material enthält.

17. Stütze nach einem der vorhergehenden Ansprüche, **dadurch gekennzeichnet, daß** der vordere Teil (1) vollständig aus einem starren, biokompatiblen Material besteht.

18. Stütze nach einem der vorhergehenden Ansprüche, **dadurch gekennzeichnet, daß** sie zusätzliche Haltemittel, die von sich in der Mitte der Stütze kreuzenden Bändern (9a, 9b) gebildet sind, sowie Bohrungen (11) in ihrer Höhe für den Durchgang der Bänder umfaßt.

19. Stütze nach einem der Ansprüche 1 bis 18, **dadurch gekennzeichnet, daß** sie zusätzliche Haltemittel umfaßt, die von unabhängigen, die Stütze in ihrer gesamten Breite durchquerenden Bändern gebildet sind.

20. Stütze nach einem der vorhergehenden Ansprüche, **dadurch gekennzeichnet, daß** der hintere Teil (2) eine Oberseite (4) umfaßt, die in ihrer Mitte und in ihrer Länge eine Nut (14) von geringer Tiefe aufweist, die geeignet ist, mit dem oberen Dorn des instrumentierten Bereichs in Kontakt zu gelangen.
